Europäisches Patentamt

European Patent Office

Office européen des brevets

(11) Publication number: **0 393 916**
**A1**

## EUROPEAN PATENT APPLICATION

(21) Application number: 90303869.3

(22) Date of filing: 10.04.90

(51) Int. Cl.5: **C12N 9/80, C12P 7/40,**
**C12N 1/20, C12S 13/00,**
**C08F 6/00, //(C12N1/20,**
**C12R1:01)**

(30) Priority: 19.04.89 GB 8908874

(43) Date of publication of application:
24.10.90 Bulletin 90/43

(84) Designated Contracting States:
BE DE DK ES FR GB IT NL

(71) Applicant: **IMPERIAL CHEMICAL INDUSTRIES**
**PLC**
**Imperial Chemical House, Millbank**
**London SW1P 3JF(GB)**

(72) Inventor: **Jones, Colin William**
**15 Ryde Avenue**
**Leicester LE2 3RS(GB)**
Inventor: **Silman, Nigel James**
**85 Carisbrooke Road**
**Leicester LE2 3PG(GB)**
Inventor: **Carver, Mark Andrew**
**Hallswood, 503 Yarm Road**
**Stockton-on-Tees, Cleveland(GB)**

(74) Representative: **Aufflick, James Neil et al**
**Imperial Chemical Industries PLC Legal**
**Department: Patents PO Box 6 Bessemer**
**Road**
**Welwyn Garden City Herts, AL7 1HD(GB)**

(54) Method for the production of amidase enzyme.

(57) A method for the production of a microorganism containing elevated levels of an amidase enzyme in which a suitable microorganism is cultivated under conditions of nitrogen limitation in a suitable medium containing an amide as a source of nitrogen at a dilution rate in the range 0.01 to 0.15 hr$^{-1}$ By an extension of the method a microorganism may be cultivated under conditions of nitrogen limitation in a medium containing as a source of nitrogen an amide which is a poor amidase substrate at a dilution rate in the range 0.01 to 0.15 hr$^{-1}$. Novel microorganisms produced by the method and processes for decomposing acrylamide and for producing acrylic acid are also claimed.

## Method for the production of amidase enzyme

This invention relates to a method for the production of amidase enzyme, (acylamide amido hydrolase E.C. No. 3-5-1-4) and to processes for using the enzyme in the decomposition of acrylamide and in the production of acrylic acid or a salt or ester thereof by decomposition of acrylamide.

Polyacrylamide polymers, i.e. homo- and hetero- polymers of acrylamide, are widely used as flocculants in the potable water industry, sewage treatment, paper manufacture and mining. However their utility is restricted and they cannot for instance be used in connection with foodstuffs because they are generally contaminated with unreacted acrylamide monomer which is a cumulative neurotoxin and a carcinogen. At present polyacrylamides are generally allowed in USA to contain up to 500 ppm of unreacted acrylamide and this limit is likely to he introduced elsewhere. This level of unreacted monomer can be achieved by use of longer polymerisation times, by a "heat treating" process and by use of sodium metabisulphate. Such treatments increase cost of manufacture and reduce the efficiency of the polymers produced by causing branching and chain scission in these polymers. Linear polymers have a higher flocculation efficiency and are preferred. There is a need for a reliable process for reducing the level of unreacted monomer in polyacrylamides without damage to the polymer to 500 ppm and preferably to even lower levels (5-10 ppm or lower). If the unreacted monomer present could be reduced reliably to very low levels (1-5 ppm or less) polyacrylamides could he considered for uses from which they are presently excluded, e.g. in the manufacture of food contact materials or in direct contact with food.

Japanese Patent Publication No. 79011389 (corresponding to Japanese kokai No. 53086078) discloses a process for the decomposition of acrylamide monomer, e.g. in waste water or in polyacrylamides by contacting with an intracellular enzyme of Brevibacterium ammoniogenes preferably of strains ATCC 1641, ATCC 6871 and ATCC 6872. However, despite being known for a number of years, this process does not appear to have been used commercially to any significant extent. Moreover Brevibacterium ammoniogenes enzyme works poorly in polyacrylamide latex systems.

Our published European Patent Specification No. 272025 describes a method for the production of an amidase enzyme wherein a bacterium belonging to the species Methylophilus methylotrophus is cultivated in a medium containing appropriate nutrients and an amide under conditions such that the amidase enzyme is induced in the bacterium.

It is desirable that amidase enzymes having increased activity should become readily available commercially and as a means of achieving this end our published European Specifcation No. 272026 describes a method for the production of an amidase enzyme of increased activity wherein microbial cells or extracts containing the enzyme are heated to a temperature in the range 40° to 80°C for a sufficient period and under such conditions that a significant increase in amidase activity is induced.

Amidases are part of the nitrogen assimilation apparatus of many cells grown under conditions where the sole source of nitrogen is an aliphatic amide.

Thus the metabolic role of amidase is to release ammonia from said amide, e.g. by the following reaction:-

$$CH_3-\overset{O}{\underset{\|}{C}}-NH_2 + H_2O \longrightarrow CH_3-\overset{O}{\underset{\|}{C}}-OH + NH_3$$

The ammonia released is then assimilated into protein biosynthesis. The aliphatic acid produced may or may not be assimilated dependent upon the microorganism type.

Amidases can also catalyse the conversion of acrylamide to acrylic acid by the following reaction:-

$$CH_2 = CH-\overset{C}{\underset{O}{\|}}-NH_2 + H_2O \longrightarrow CH_2 = CH-\overset{C}{\underset{O}{\|}}-OH + NH_3$$

In the presence of appropriate salts or alcohols the acrylic acid produced can he converted to its salt or ester.

Using the methods of European Patent Specifications Nos. 272025 and 272026 it is possible to provide amidase enzymes for use in processes for the decomposition of acrylamide monomer which exhibit activities in the range 1 fo 3.5 units (mols per minute per mg), typically approximately 2 units. However still further increases in activity for amidase enzymes for use in such processes are desirable.

According to the present invention we provide a first method for the production of a microorganism containing elevated levels of an amidase enzyme using a directed chemostat selection wherein a microorganism containing amidase enzymes or in which such enzymes can be induced is cultivated under conditions of nitrogen limitation in a suitable growth medium containing an amide as a source of nitrogen at a low dilution rate in the range 0.1 to 1.15 $hr^{-1}$ for a period sufficient to increase substantially the levels of amidase activity seen.

Further according to the present invention we provide a second method for the production of a microorganism containing elevated levels of an amidase enzyme of improved kinetic properties using a directed chemostat selection wherein a microorganism containing amidase enzymes or in which such enzymes can be induced or a microorganism which contains elevated levels of amidase enzymes produced by the first method of the invention is cultivated under conditions of nitrogen limitation in a suitable growth medium containing, as a source of nitrogen, an amide which is a poor amidase substrate, at a low dilution rate in the range 0.01 to 0.15 hr$^{-1}$ for a period sufficient to increase substantially the levels of amidase activity seen.

Microorganisms containing elevated levels of an amidase enzyme produced by the first and second methods of the invention also form part of the invention.

Further according to the present invention we provide a process for the decomposition of acrylamide in a medium containing it in which the medium is contacted with an enzyme capable of decomposing acrylamide under conditions suitable for the enzyme to decompose the acrylamide wherein the enzyme is an amidase enzyme which has been produced by the first or second method of the invention.

Further according to the invention we provide a process for the production of acrylic acid or a salt or ester thereof in which a medium containing acrylamide is contacted with an enzyme capable of decomposing acrylamide to acrylic acid under conditions suitable for the enzyme to decompose the acrylamide to acrylic acid or a salt or ester thereof wherein the enzyme is an amidase enzyme which has been produced by the first or second method of the invention.

Preferably the poor amidase substrate which is used in the second method of the invention is acrylamide.

The decomposition process of the invention can be used to decompose acrylamide in any medium in which it occurs. It is particularly useful for decomposing unreacted acrylamide present in polyacrylamide polymers and acrylamide present in waste waters, e.g. waste water from a process for the production of acrylamides or polyacrylamides.

Polyacrylamides are produced as polymers of three chemical types. These are cationic, anionic and nonionic polymers and include copolymers wherein acrylamides may be copolymerised with other monomers, e.g. acrylic acid. These polymers may be manufactured by one of the following three basic technologies:-

Solution polymerisation wherein monomers are polymerised in aqueous solution to produce a solution product;

Dry polymers which are polymers produced as above but which are subsequently heat dehydrated prior to use;

Latex or suspension polymerisation wherein a solution of monomers in water is admixed with detergents and a non-aqueous solvent, typically low odour paraffins to form a stable suspension of aqueous droplets within which beads of polymers are formed by addition of a polymerisation initiator to the system.

Latex or suspension polymers form the largest group of polyacrylamides in terms of market share.

Anionic and neutral polyacrylamide polymers are readily treated by direct addition of amidase using the process of the invention or other processes, e.g. the process of our European Patent Specification No. 272026 since they are formulated to have pH values in the range 6 to 9. Cationic polyacrylamide polymers may also be treated with amidase by the process of the invention or by other processes but these polymers are formulated to have acid ph values in the range 3 to 4 and treatment by direct addition of amidase is less effective than in the case of anionic and neutral polymers.

Cationic polyacrylamide polymers are readily treated by a process having the following steps:

(A) raising the pH of the polymer to be treated from a value in the range 3 to 3.5 to a value in the range 4 to 9; and thereafter

(B) treating the polymer with the enzyme under conditions suitable for the enzyme to decompose the acrylamide.

The amidase enzyme used in the decomposition and acrylic acid production processes of the invention may he present in any suitable form in whole microorganism cells or as a crude or purified enzyme extract. The enzyme can he introduced to the processes in whole cells in the culture produced initially in the method of the invention or in a medium produced after only partial separation of water and other components from such a culture.

Generally however it is preferred that the cells should he separated from the culture before being used in the processes.

Preferably the methods of the invention are used for the production of bacteria containing elevated levels of an amidase enzyme capable of decomposing acrylamide which may be used in the processes of the invention. Hereinafter the invention will be described mainly in terms of the production and use of this enzyme.

Preferably the first and second methods of the invention are used for the production of bacteria containing amidase enzyme of improved kinetic properties, higher turnover numbers, improved Michaelis constant (Km) from strains of the species Methylophilus methlyotrophus. This species (formerly named Pseudomonas methylotropha), strains of which are cultivated under the conditions of the methods, is described in our UK Patent Specification No. 1370892. Strains of this species suitable for use in the methods have been deposited at the following 3 culture collections from which cultures are available:-

1. The National Collections of Industrial and Marine Bacteria Ltd (NCIMB), 23 St Machar Drive, Aberdeen AB2 1RY, Scotland, UK;

2. The Agricultural Research Culture Collection (NRRL), 1815 North University Street, Peoria, Illinois 61604, USA; and

3. The Fermentation Research Institute (FRI), Agency of Industrial Science and Technology, Ministry of International Trade and Industry, 1 - 3 Higashi 1-Chome, Yatabe-machi, Tsukuha-gun, Ibaragi-ken, Japan.

The corresponding accession numbers assigned to the strains deposited at these collections are as follows:-

NCIB 10508 - 10515 & 10592 - 10596, all inclusive;

NRRL B 5352 - B 5364 inclusive; and

FRI 1215 - 1227 inclusive.

A preferred strain for use in the first and second methods of the invention is Methylophilus methylotrophus strain AS1 (NCIB 10515) which may safely be used in treating, e.g. polyacrylamide polymers intended for use in connection with foods. The species Methylophilus methylotrophus and the above strains, particularly strain AS-1, have become widely known and are mentioned in numerous publications both by us and others. In addition to the deposits mentioned above cultures of them are also held in a number of University and other laboratories in the UK and in other countries. Also very suitable for use in the methods of the invention is strain NCIB 11585, available from NCIB, the production of which by the genetic modification of strain NCIB 10515 is described in our European Patent Specification 35831 B and a number of other publications.

In the methods of the invention bacterial cells are cultivated aerobically in a medium containing sources of carbon, nitrogen, phosphorus and other appropriate nutrients with an amide being present under conditions such that amidase is induced in the bacterial cells. Suitably cultivation takes place at a temperature in the range 20° to 40°C, preferably 34° to 38°C, and at a pH in the range 5.0 to 8.0, preferably 5.8 to 7.6. Methanol is the preferred carbon source. Any suitable amide may be present in the culture medium during the first method of the invention although acetamide is preferred. Other suitable amides for the first method include amides of carboxylic acids having the formula:-

$$R- \underset{\underset{O}{\|}}{C} -OH$$

Where R is a short chain aliphatic group, i.e. containing 1 to 5 carbon atoms, which may be a straight or a branched chain. In the methods of the invention the amide is preferably the sole or major nitrogen source. A very suitable culture medium for the methods has the following composition:-

| Component | Amount present/litre |
|---|---|
| Phosphoric acid : | 1.6 ml |
| $MgSO_4.7H_2O$ : | 1.912 g |
| $K_2SO_4$ : | 0.952 g |
| $CuSO_4.5H_2O$ : | 0.840 mg |
| $ZnSO_4.H_2O$ : | 2.568 mg |
| $MnSO_4.4H_2O$ : | 4.04 mg |
| $FeSO_4.7H_2O$ : | 37.20 mg |
| Calcium formate : | 0.173 g |

Nitrogen is supplied either from the amide alone or from the amide and ammonia. Cells are grown in nitrogen limitation at a range of cell concentrations.

Cells produced can be harvested by any suitable means preferably by ultrafiltration or centrifugation to produce a slurry having 10 -25% by weight dry solids. Suitably this slurry is broken, e.g. by several freeze-

4

thaw cycles or by mechanical breakage in a bead mill or french pressure device. Cell debris may then be removed by centrifugation leaving a crude cell-free extract containing amidase. Heat treatment may take place before or after this centrifugation but preferably before as this gives improved sedimentation of the product during centrifugation in addition to stimulating activity. This extract can if desired be further purified by anionic ion exchange chromatography and gel filtration. The cell free amidase preparations are suitably stored cool at 0° -10°C or as frozen solutions or as freeze dried preparations prior to hydration and use.

When cells or cell free extracts containing amidase produced by the methods of the invention are heated to temperatures in the range 40° to 80°C, as described in our European Patent Specification No. 272,026 we have found that, most usually, the amidase activity is irreversibly increased by 1.5 to 35 times dependent upon the enzyme preparation. This effect is greatest at temperatures in the range 55°to 65°C, especially in the range 58° to 62°C, at pHs in the range 4 to 9, especially at pHs between 6 and 7 and at protein concentrations in the range 0.1 to 200 mg/ml. Under these conditions little significant denaturation of amidase takes place. Heating suitably takes place for a period in the range 20 minutes to 10 hours, especially 30 minutes to 180 minutes in some instances. To produce the increased activity the cells or cell-free enzyme can be heated immediately after they have been produced by the methods of the invention or at some later time. The methods of the invention can usefully be carried out either as described above or as a similar process having four steps, i.e. a first fermentation step in which cells having induced amidase are grown in a fermenter, a second centrifugation step wherein cells are concentrated to a 10 to 20% dry solids slurry, a third heat shock step in which cells are subjected to heating to a temperature in the ranges described above and a fourth separation step in which a precipitate containing debris is separated from a supernatant liquid containing the enzyme.

In the process of the invention for decomposing acrylamides amidase can be used to reduce free acrylamide residues occurring in all types of acrylamide polymers, particularly latices of the three basic chemical types. When treating a latex an amidase solution is added to the latex and is dispersed through the latex by stirring or similar mixing techniques at a level between 1 and 10,000 units/kg latex preferably 100 - 2000 U/kg latex. Incubation of amidase with the latex results in conversion of free acrylamide to acrylic acid or a salt thereof. Incubation temperatures of 10°C to 100°C (particularly 30° to 80°C) are preferred. The latices are are suitably treated over a pH range 3 to 10, preferably 5 to 7.

The amidase produced in the method of the invention converts acrylamide into acrylic acid. This reaction can therefore be used as a means for producing acrylic acid or, when carried out in the presence of a suitable salt or alcohol, to produce salts or esters of acrylic acid.

The amidase produced by the method of the invention particularly when heated as described above exhibits very high activity. Thus use of the process of the invention for decomposing acrylamide greatly extends the range of utility of polyacrylamide polymers.

The first and second methods of the invention are illustrated by the following Examples:-

Example 1

Microorganisms growing in nutrient limitation in continuous culture can be put under considerable directed selective pressure by growth at very low dilution rate as there the standing concentration of the limiting nutrient is very low. Organisms that have improved assimilation of this limiting nutrient will be strongly selected for use in such a regime. Such improved assimilations may be caused by production of higher levels of the enzyme involved in the primary metabolism of the limiting nutrient.

We have found that when cultivation of cells takes place under the conditions of the first method of the invention microorganism strains containing elevated levels of amidase enzymes are obtained.

Suitably during cultivation cells of a parent strain Methylophilus methylotrophus AS 1 containing amidase or in which amidase can be induced are transferred from batch culture to continuous culture in nitrogen limitation in the presence of an amide, e.g. acetamide as a nitrogen source and cultivated in continuous culture at very low dilution rate in the range 0.01 to 0.15 $h^{-1}$ particularly 0.025 $h^{-1}$. After a period of growth at this dilution rate, e.g. a period within the range 200-500 hours, directed selection pressure will select for cells containing elevated levels of amidase activity. After this period the dilution rate is raised to a second dilution rate in the range 0.05 to 0.55 $h^{-1}$ particularly 0.1 $h^{-1}$ and held at this level for 100 to 500 hours. After this single organisms are isolated in a sterile manner and regrown under conditions of N limitation in the presence of an amide as nitrogen source at a dilution rate of 0.1 $h^{-1}$. Organisms having elevated levels of amidase activity compared to the parent strain grown under similar conditions are selected and can be used in the processes of the invention. A strain produced by this procedure is Methylophilus methylotrophus strain MM6 (see Table below).

Example 2

Directed selection in chemostat culture can also be used to improve the catalytic efficiency of an enzyme if the correct selection pressure can be exerted.

We have found that when cultivation of cells takes place under the conditions of the second method cells containing elevated levels of amidase of an improved catalytic efficiency are obtained. Suitably during cultivation cells of a parent strain which is Methylophilus methyltrophus AS1, containing amidase or in which amidase can be induced, or which is preferably a strain produced by the first method of the invention such as Methylophilus methylotrophus MM6, are transferred from batch culture to continuous culture in nitrogen limitation in the presence of an amide that is a poor substrate for the enzyme e.g. acrylamide as a nitrogen source and are cultivated in continuous culture at a very low dilution rate in the range 0.01 to 0.15 $h^{-1}$ particularly 0.025 $^{-1}$. After a period of growth at this dilution rate, e.g. a period within the range 200-500 hours, directed selection pressure will select for containing elevated levels of amidase of improved catalytic cells efficiency (either increased turnover number or decreased Km or both).

After this period the dilution rate is raised to a second dilution rate in the range 0.05 to 0.55 $h^{-1}$, particularly 0.1 $h^{-1}$, and held at this level for 100-500 hours. After this, single organisms are isolated in a sterile manner and regrown under conditions of nitrogen limitation in the presence of an amide as nitrogen source at a dilution rate of 0.1 $h^{-1}$. Organisms having elevated levels of amidase activity of improved catalytic efficiency compared to the parent strain (i.e. either AS1 or MM6) are selected and and used in the methods of the invention. A strain produced by this procedure is Methylophilus methylotrophus MM8 (see Table below).

Strains MM6 and MM8 respectively exhibit the properties relative to the basic AS-1 strain which are shown in the Table below.

| Strain | Turnover Number K cat sec $^{-1}$ | Km mM | K cat/Km | % Total Protein which is amidase |
|---|---|---|---|---|
| AS1 NCIB 10515 | 96 | 18 | 5.3 | 4 |
| MM6 | 96 | 10.18 | 5-->6 | 25 |
| MM8 | 320 | 12 | 2.7 | 26 |

The Table shows that and MMB are microorganisms in which the proportion of the total protein which is present as amidase is very high. The Michaelis Constant Km represents the concentration of substrate which allows the enzyme to function at half its maximum velocity.

Strains MM6 and MM8 were deposited under the terms of the Budapest Treaty on the International Recognition of the Deposit of microorganisms for the Purposes of Patent Procedure on 23 August 1989 at the National Collections of Industrial and Marine Bacteria Ltd (NCIMB), 23 St Machar Drive, Aberdeen AB2 1RY, Scotland, Uk and have been given the accession numbers NCIMB 40181 and NCIMB 40182 respectively.

**Claims**

1. A method for the production of a microorganism containing elevated levels of an amidase enzyme using a directed chemostat selection wherein a microorganism containing amidase enzymes or in which such enzymes can be induced is cultivated under conditions of nitrogen limitation in a suitable growth medium containing an amide as a source of nitrogen at a low dilution rate in the range 0.1 to 1.15 $hr^{-1}$ for a period sufficient to increase substantially the levels of amidase activity seen.

2. A method for the production of a microorganism containing elevated levels of an amidase enzyme of improved kinetic properties using a directed chemostat selection wherein a microorganism containing amidase enzymes or in which such enzymes can be induced or a microorganism which contains elevated levels of amidase enzymes produced by the method of claim 1 is cultivated under conditions of nitrogen limitation in a suitable growth medium containing, as a source of nitrogen, an amide which is a poor amidase substrate, at a low dilution rate in the range 0.01 to 0.15 $hr^{-1}$ for a period sufficient to increase substantially the levels of amidase activity seen.

3. A method according to claim 2 wherein the poor amidase substrate is acrylamide.

4. A method according to any one of claims 1 to 3 wherein the microorganism is a strain of the species .

Methylophilus methylotrophus.

5. A method according to claim 4 wherein the strain is Methylophilus methylotrophus AS 1 (NCIB 10515).

6. A method according to any one of the preceding claims wherein the microorganism is cultivated at a temperature in the range 34° to 38°C.

7. A method according to any one of the preceding claims wherein the microorganism is cultivated at a pH in the range 5.8 to 7.6.

8. A method according to any one of the preceding claims wherein the microorganism or a cell free extract thereof containing amidase which has been produced by cultivation is thereafter heated to a temperature in the range 55° to 65°C at a pH in the range 6 to 7 and at a protein concentration in the range 0.1 to 200 mg/ml.

9. Microorganisms containing elevated levels of an amidase enzyme which have been produced by a method according to any one of claims 1 to 8.

10. Methylophilus methylotrophus strains NCIMB 40181 and NCIMB 40182 and variants and mutants derived therefrom.

11. A process for the decomposition of acrylamide in a medium containing it in which the medium is contacted with an enzyme capable of decomposing acrylamide under conditions suitable for the enzyme to decompose the acrylamide wherein the enzyme is an amidase enzyme which has been produced by a method according to any one of claims 1 to 8.

12. A process according to claim 11 for decomposing unreacted acrylamide present in polyacrylamide polymers or acrylamide present in waste waters.

13. A process for the production of acrylic acid or a salt or ester thereof in which a medium containing acrylamide is contacted with an enzyme capable of decomposing acrylamide to acrylic acid under conditions suitable for the enzyme to decompose the acrylamide to acrylic acid or a salt or ester thereof wherein the enzyme is an amidase enzyme which has been produced by a method according to any one of claims 1 to 8.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | EP-A-272026 (IMPERIAL CHEMICAL INDUSTRIES PLC) <br> * page 3, lines 30 - 52; claims * <br> * page 4, line 10 - page 5, line 5 * <br> * page 5, line 33 - page 42 * <br> & A- <br> --- | 1, 4-9, 11-13 | C12N9/80 <br> C12P7/40 <br> C12N1/20 <br> C12S13/00 <br> C08F6/00 <br> //(C12N1/20, <br> C12R1:01) |
| A | PATENT ABSTRACTS OF JAPAN <br> vol. 11, no. 13 (C-397)(2460) 14 January 1987, <br> & JP-A-61 187788 (DAICEL CHEM. IND. LTD.) 21 August 1986, <br> * the whole document * <br> --- | 2 | |
| P,X | J. GEN. MICROBIOL. <br> vol. 135, 1989, <br> pages 3153 - 3164; SILMAN, N.J. et al.: <br> "Physiology of amidase production by Methylophilus methylotrophus: Isolation of hyperactive strains using continuous culture." <br> * the whole document * <br> ----- | 1-13 | |
| | | | **TECHNICAL FIELDS SEARCHED (Int. Cl.5 )** <br><br> C12N <br> C12P <br> C12K |

The present search report has been drawn up for all claims

| Place of search | Date of completion of the search | Examiner |
|---|---|---|
| THE HAGUE | 16 JULY 1990 | ANDRES S.M. |

EPO FORM 1503 03.82 (P0401)